# EUROPÄISCHE PATENTSCHRIFT

(11) **EP 1 613 354 B1**
(45) Veröffentlichungstag und Bekanntmachung des Hinweises auf die Patenterteilung: **20.08.2008**
(21) Anmeldenummer: 04723211.1
(22) Anmeldetag: 25.03.2004
(51) Int. Cl.: A61K 47/08, A61K 47/22

(54) **VERWENDUNG VON CYCLISCHEN ACETALEN/KETALEN ZUR VERBESSERTER PENETRATION VON WIRKSTOFFEN IN ZELLEN UND ORGANEN**
USE OF CYCLIC ACETALS/ CETALS FOR IMPROVED PENETRATION OF ACTIVE SUBSTANCES INTO CELLS AND ORGANS
UTILISATION DES ACETALS/CETALS CYCLIQUES POUR UNE PENETRATION AMELIOREE D'AGENTS ACTIFS DANS DES CELLULES ET DES ORGANES

(30) Priorität: 02.04.2003 DE 10314976
(43) Veröffentlichungstag der Anmeldung: 11.01.2006
(73) Patentinhaber: Bayer HealthCare AG, 51368 Leverkusen (DE)
(72) Erfinder: HARDER, Achim, 51109 Köln (DE); HEEP, Iris, 50859 Köln (DE); HERRMANN, Stefan, 40764 Langenfeld (DE); GRUNKEMEYER, Jeffry-Lynn, CH-4133 Pratteln (CH); KALBE, Jochen, 42799 Leichlingen (DE); MEHLHORN, Heinz, 41468 Neuss (DE); SCHMIDT, Jürgen, 40221 Düsseldorf (DE); SCHMAHL, Günther, 50735 Köln (DE)
(86) Internationale Anmeldenummer: PCT/EP2004/003155
(87) Internationale Veröffentlichungsnummer: WO 2004/087117

(56) Entgegenhaltungen:
- EP-A- 0 268 460
- EP-A- 0 552 879
- EP-A- 0 728 462
- WO-A-99/09954
- WO-A-99/20257
- WO-A-03/028702
- US-A- 3 004 894
- XIAOYING HUI ET AL: "Enhanced Econazole Penetration into Human Nail by 2-n-Nonyl-1,3-dioxolane" JOURNAL OF PHARMACEUTICAL SCIENCES, Bd. 92, Nr. 1, Januar 2003 (2003-01), Seiten 142-148, XP002305669
- FUHRMAN L C ET AL: "Effect of novel penetration enhancers on the transdermal delivery of hydrocortisone: an in vitro species comparison" JOURNAL OF CONTROLLED RELEASE, ELSEVIER SCIENCE PUBLISHERS B.V. AMSTERDAM, NL, Bd. 45, Nr. 2, 17. März 1997 (1997-03-17), Seiten 199-206, XP004559396 ISSN: 0168-3659

## Beschreibung

Die vorliegende Erfindung betrifft die Verwendung von bestimmten cyclischen Acetalen oder Ketalen zur Verbesserung der Penetration von pharmazeutischen Wirkstoffen in Zellen und Organen.

Larvenstadien von Helminthen, adulte Helminthen, Protozoen, eine Reihe von Mikroorganismen (Bakterien, Pilze, Viren usw.) sowie Tumore befallen innere Organe des Menschen und der Tiere, z.B. Gehirn, Leber, Muskeln usw. Hier wirken die gängigen Anthelminthika, Antiprotozoika, Antibiotika, Virostatika, Chemotherapeutika, Impfstoffe und Modulatoren der spezifischen und unspezifischen Immunabwehr etc. oftmals nur in hohen Dosierungen bzw. gar nicht. Die Schwierigkeit besteht darin, dass Zell- bzw. Organbarrieren, wie z.B. Zellmembranen, die Bluthirnschranke und die Placentaschranke, für die verschiedenartigsten Chemotherapeutika wenig durchgängig sind.

Die Verwendung von Dialkyldioxolanen als grenzflächenaktive Stoffe (Surfactants) ist bereits bekannt (siehe z. B. Wo 00/70334 oder US 3 948 953).

WO 01/17345 beschreibt die Verwendung von bestimmten Acetalen bzw. Ketalen zur Herstellung von biologisch abbaubaren Lösungen biologisch aktiver Verbindungen, und zwar von Herbiziden, Safenern, Insektiziden, Fungiziden, Akariziden, Nematiziden, Pheromonen und Repellents.

WO 99/09954 beschreibt die Verwendung von 2-Nonyl-1,3-dioxolan und anderen Hydrocarbyl-Derivaten von 1,3-Dioxolan oder 1,3-Dioxan zur Verbesserung der Hautpenetration bei der dermalen Applikation von Arzneimitteln. Als Wirkstoffe werden Ibuprofen und andere NSAIDs genannt.

Michniak et al. beschreiben in Drug Delivery (2), (1995), 117-22, in-vitro-Untersuchungen an Haut von haarlosen Mäusen mit dem Ziel, für Derivate von 2-(1-Nonyl)-1,3-Dioxolan Struktur-Wirkungs-Beziehungen für die Verbesserung der Hautpenetration von Hydrocortison (als Modell) aufzustellen.

Phillips et al., beschreiben in J. Pharm. Soci. 84 (12) (1995), 1427-33 Untersuchungen zur transdermalen Verabreichung von Arzneimitteln mit unterschiedlicher Lipophilie unter Verwendung von Azone-Analoga als Enhancer der Hautpenetration. Eine der getesteten Verbindungen war 2-(1-Nonyl)-1,3-dioxolan.

EP 268 460A1 offenbart neue 1,3-Dioxacycloalkane, die sich als Absorptionsenhancer für die dermale Applikation eignen.

WO 99/20257 beschreibt die Verwendung von 2-n-Nonyl-1,3-dioxolan und andere Kohlenwasserstoffderivate des 1,3-Dioxolans in Formulierungen zur topischen Anwendung auf der Haut, die bei der Hormonersatztherapie eingesetzt werden sollen.

WO 03/028702 betrifft Cremeformulierungen des Ibuprofens, die einen Enhancer für die Hautpenetration wie z. B. 2-n-Nonyl-1,3-dioxolan oder Decanaldimethylacetal enthalten können.

EP 0 552 879A1 befasst sich mit der Verbesserung der lontophorese mittels bestimmter Verbindungen, die auf die Haut aufgebracht werden und die transdermale Verabreichung des Wirkstoffs verbessern sollen.

US 3 004 894 offenbart ein Arzneimittel, das ein Tetracyclin sowie ein Dioxolan enthält, wobei sich das Mittel besonders zur parenteralen Verabreichung eignet.

Hui et al., Journal of Pharmaceutical Sciences, 92, (2003), 142-148 offenbart die Verwendung von 2-n-Nonyl-1,3-dioxolan zur Verbesserung der Penetration von Econazol in den menschlichen Nagel.

Fuhrmann et al., Journal of Controlled Release, 45, (1997), 199-206 befasst sich mit der Wirkung von bestimmten neuen Penetrationsenhancern auf die transdermale Verabreichung von Hydrocortison.

EP 0 728 462A2 offenbart ein Verfahren und Mittel zur Behandlung von Haarausfall, wobei das Mittel eine 1,3-Dioxolan-Verbindung enthalten kann.

Erdlenbruch et al. untersuchten (Exp. Brain Res. 135 (2000) 417-422) die vorübergehende und kontrollierbare Öffnung der Bluthirnschranke für cytostatische und antibiotische Mittel bei Ratten. Sie verwendeten jedoch Alkylglycerole und applizierten diese intraarteriell in die Carotis-Arterie.

Nach wie vor besteht Bedarf an Penetrationsenhancern, welche die Durchlässigkeit von Zell- und Organbarrieren für Arzneimittel verbessern, so dass diese an den Ort des Krankheitsgeschehens gelangen können. Die Arzneimittel würden dadurch entweder schneller oder in geringeren als den üblichen Dosierungen wirken können, auch könnten bislang unwirksame Verbindungen durch den Zusatz solcher die Penetration verbessernden Verbindungen erst wirksam werden.

Überraschenderweise wurde nun gefunden, dass dieses Ziel mit Hilfe bestimmter cyclischer Acetale erreicht werden kann. Die Erfindung betrifft daher die

Verwendung von Verbindungen der Formel (I) 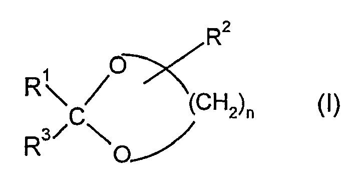 worin
- R¹: für einen gegebenenfalls mit einem oder mehreren Halogenatomen substituierten Alkyl-, Alkenyl- oder Alkinylrest mit 2 bis 30 Kohlenstoffatomen steht, wobei gegebenenfalls ein oder mehrere geeignete, nicht benachbarte Kohlenstoff-Kettenglieder durch Sauerstoffatome ersetzt sein können,
- R²: für Wasserstoff, Hydroxyl, -NH₂, -NR⁴R⁵, -N⁺(R⁴R⁵R ⁶), -PR⁷R⁸, -O-P(R⁷R⁸), -P(O)R⁷R⁸, -P⁺(R⁷R⁸R⁹) oder einen C₁₋₅-Alkylrest steht, der gegebenenfalls durch Hydroxyl, C₁₋₄-Alkoxy, -NH₂, Mono- oder Di-C₁₋₄-alkylamino oder einen 5- bis 7-gliedrigen Heterocyclus mit bis zu drei Heteroatomen, ausgewählt aus O, N und S, substituiert ist,
- R³: für Wasserstoff steht oder die oben angegebenen Bedeutungen von R¹ annehmen kann,
- R⁴, R⁵ und R⁶: unabhängig von einander für Wasserstoff oder C₁₋₅-Alkyl stehen oder zwei der Reste gemeinsam mit dem Stickstoffatom, an das sie gebunden sind, einen 5- bis 7-gliedrigen Heterocyclus bilden, der gegebenenfalls noch ein oder zwei weitere Heteroatome, ausgewählt aus O, N und S, enthalten kann,
- R⁷, R⁸ und R⁹: unabhängig von einander für C₁₋₅-Alkyl, C₁₋₅-Alkoxy oder C₆₋₁₂-Aryl stehen
oder
zwei der Reste gemeinsam mit dem Phosphoratom, an das sie gebunden sind, einen 5-7-gliedrigen Heterocyclus bilden, der gegebenenfalls noch ein oder zwei weitere Heteroatome, ausgewählt aus O, N und S, enthalten kann.
- n: 2, 3 oder 4 bedeutet,
zur Herstellung von Arzneimitteln mit verbessertem Durchtritt eines pharmazeutischen Wirkstoffs durch Zell- und Organbarriren, wobei das Arzneimittel oral verabreicht wird

Alkylreste können im allgemeinen geradkettig oder verzweigt sein. Dies gilt auch für Alkylreste in anderen Substituenten, wie z. B. Alkoxy oder Alkylamino.

Alkenyl- und Alkinylreste können ebenenfalls geradkettig oder verzweigt sein und enthalten je nach Kohlenstoffzahl eine oder mehrere Doppel- bzw. Dreifachbindungen, vorzugsweise enthalten Alkenyl- und Alkinylreste eine Doppel- bzw. Dreifachbindung.

C₆₋₁₂-Aryl steht für einen carbocyclischen aromatischen Rest mit 6 bis 12 Kohlenstoffatomen, z. B. Phenyl oder Naphthyl.

Heterocyclus kann z. B. stehen für: Pyrrolidin, Piperidin, Piperazin, Hexamethylenimin, Hexahydro-1,3,5-triazin, Morpholin.
- R¹: steht bevorzugt für einen gegebenenfalls durch ein oder mehrere Fluor- oder Chloratome substituierten n-Alkylrest mit 2 bis 20 Kohlenstoffatomen, in dem eines der Kohlenstoff-Kettenglieder durch ein Sauerstoffatom ersetzt sein kann.
- R¹: steht besonders bevorzugt für einen n-Alkylrest mit 5 bis 12 Kohlenstoffatomen

Falls R¹ für einen Alkylrest steht, der durch ein oder mehrere Sauerstoffatome unterbrochen sein kann, so ist bevorzugt der Acetalring über ein Kohlenstoffatom des Restes R¹ angeknüpft.
- R²: steht bevorzugt für Hydroxyl, C₁₋₃-Alkyl, das gegebenenfalls mit Hydroxyl oder C₁₋₅-Alkoxy substituiert ist.
- R²: steht besonders bevorzugt für OH oder -CH₂OH.
- R³: steht bevorzugt für Wasserstoff.
- n: steht bevorzugt für die Zahlen 2 oder 3.

Die Verbindungen der Formel (I) können gegebenenfalls in Abhängigkeit von der Art und Anzahl der Substituenten als geometrische und/oder optische Isomere oder deren Isomerengemische in unterschiedlicher Zusammensetzung vorliegen. Sowohl die reinen Isomeren als auch die Isomerengemische können erfindungsgemäß verwendet werden.

Weiterhin können auch Gemische verschiedener Verbindungen der Formel (I) erfindungsgemäß verwendet werden.

Die erfindungsgemäß verwendeten Verbindungen der Formel (I) können auch in Form pharmazeutisch akzeptabler Salze eingesetzt werden, vorausgesetzt sie tragen entsprechende Substituenten. Pharmazeutisch akzeptable Salze können Salze mit anorganischen oder organischen Säuren sein, wie z. B. Chlorwasserstoffsäure, Essigsäure oder Maleinsäure. Pharmazeutisch akzeptable Salze können ebenso Salze mit Basen sein, wie beispielsweise Metall- oder Ammoniumsalze, z. B. Alkalimetallsalze, wie Natrium- oder Kaliumsalze, oder Ammoniumsalze beispielsweise mit Triethylamin, Triethanolamin oder Arginin.

Die Verbindungen der Formel (I) und ihre Herstellung sind dem Fachmann bekannt, gegebenenfalls kann die Herstellung analog zu bekannten Verfahren erfolgen. Hierzu wird z.B. auf WO 01/17345 oder Tenside Detergents 1980, 17,21-24 verwiesen.

Beispiele für besonders bevorzugt verwendbare Verbindungen sind die folgenden Verbindungen der Formeln (A) und (B)

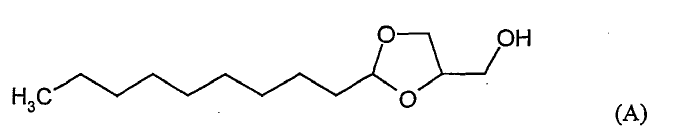

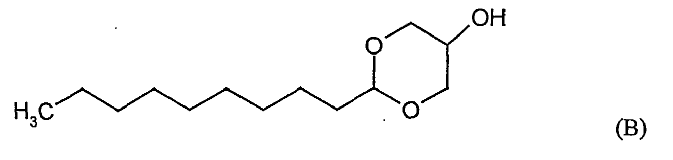

Die erfindungsgemäß verwendeten Verbindungen der Formel (I) eignen sich prinzipiell zur Verbesserung der Penetration von Zell- und Organbarrieren für die verschiedenartigsten Arzneimittel, bei denen dies nötig oder sinnvoll ist. Beispielsweise seien genannt: Anthelminthika, Antiprotozoika, Antibiotika, Virostatika, Chemotherapeutika, Impfstoffe, Modulatoren der spezifischen und unspezifischen Immunabwehr sowie Arzneimittel zur Behandlung von ZNS-Erkrankungen. Im folgenden sind Beispiele von Erkrankungen bei Mensch und Tier zusammen mit beispielhaft genannten Wirkstoffen zu deren Behandlung aufgelistet, bei denen die erfindungsgemäße Verwendung von Verbindungen der Formel (I) vorteilhaft ist:

### 1. Parasiten (Mensch)

| | | |
|---|---|---|
| a) | Toxoplasma, z. B. T. gondii (Hirn) | : Pyrimethamin/Sulfadiazin |
| b) | Chagas-Krankheit (Herz, Nerven) | : NifurtimoxBenznidazol |
| c) | Pneumocystis carinii (Lunge) | : Cotrimoxazol/Trimethoprim |
| d) | Filarien (Gewebe) | : Diethylcarbamazin, makrocyclische |
| | | Lactone (Avermectine, Milbemycine) |
| e) | Trichinen (Muskel) | : Mebendazol, Thiabendazol |
| f) | Schlafkrankheitserreger (Hirn) | : Suramin, Melarsoprol, Eflornithin |
| g) | Leishmaniose-Erreger (Haut, innere | : 5-wertige Antimonpräparate, Pentamidine, |
| | Organe) | Megluminantimoat |
| h) | Hirnwurm (Angiostrongylus) | : Me- und Thiabendazol, makrocyclische |
| | | Lactone (z. B. Avermectine, Milbemycine), |
| | | Depsipeptide (z. B. Emodepsid, PF 1022A) |
| i) | Toxocara-Wanderstadien im | : Me- und Thiabendazol |
| | Körper/Larva migrans: | |
| j) | Echinococcus-Zysten (Leber) | : Me- und Albendazol stoppt Wachstum |
| k) | Amoebenabzeß in der Leber | : Nitroimidazole, Dehydroemetin, |
| | | Paromomycin |
| 1) | Mikrosporidien | : Albendazole |
| m) | Trematoden | : Praziquantel |
| n) | Bandwurm-Zystizerken | : Praziquantel |

### 2. Parasiten (Tier)

| | | |
|---|---|---|
| a) | Toxocara-Larven im Muttertier | : Benzimidazole (Bendazole) |
| b) | Kokzidien (Hühner/Rinder; Darm) | : Toltrazuril, Ponazuril, Ionophoren |
| c) | Nematoden (Darm) | : Breites Spektrum von Anthelminthika |
| | | und deren Kombinationen (z. B. |
| | | Febantel, Pyrantel) |
| d) | Fasciola hepatica | : Salicylanilide, Triclabendazol, Clor sulon, |
| | | Diamfenetid, Praziquantel |
| e) | Trichine (Muskulatur) | : Me- und Thiabendazole |
| f) | Cryptosporidien | : Halofuginon |
| g) | Hammondia/Neospora/Toxoplasma | : Toltrazuril |
| h) | Zystizerken, Spargana von Bandwürmern : | Praziquantel |
| i) | adulte Bandwürmer (Darm) | : Niclosamid/Praziquantel |
| j) | Leishmaniose-Erreger (Hund) | : Pentamidine, Antimonverbindungen |
| k) | Herzwurm/Dirofilaria (Hund) : makrocyclische Lactone (Avermectine, | |
| | | Milbemycine), Depsipeptide (Emodepsid, |
| | | PF 1022 A) |
| i) | Erreger der Räude, Demodikose (Hund, : | makrocyclische Lactone (Avermectine, |
| | Katze, Schwein) | Milbemycine) |

### 3. Bakterien (Mensch/Tier)

| | | |
|---|---|---|
| a) | Hirn (z.B. Meningococcen) | Antibiotika |
| b) | Lunge (z.B. Pneumococcen) | Antibiotika |
| c) | Herz/Darm (z.B. Helicobacter, | Antibiotika |
| | Chlamydien) | |
| d) | Nerven (z.B. Borrelia burgdorferi) | Antibiotika |

### 4. Karzinome (Mensch/Tier)

| | | |
|---|---|---|
| a) | Hirn, Lunge, Leber, Knochen etc. | Cytostatika |

### 5. ZNS-Erkrankungen

| | | |
|---|---|---|
| a) | psychische Störungen | z.B. Antidepressiva |
| b) | Alzheimer'sche Krankheit | |
| c) | Parkinson'sche Erkrankung | |
| d) | Schizophrenie | |

### 6. Schlaganfall (Stroke)

Die Verbesserung der Penetration von Zell- und Organbarrieren bedeutet insbesondere ein verbessertes Überwinden der Bluthirnschranke, der Placentaschranke sowie verbesserte Penetration in Zellen der Muskeln und der Leber. Mit den erfindungsgemäßen Verbindungen bietet sich somit erstmals die Möglichkeit - beispielsweise auch bei oraler Verabreichung üblicher Dosen an Wirkstoff - in Organen wirksame Wirkstoffkonzentrationen zu erreichen, in denen dies sonst kaum zu erreichen ist. Als wichtiges Beispiel sei die sehr schlechte Durchlässigkeit der Bluthirnschranke für viele der oben genanten Wirkstoffe genannt.

Als Wirkstoffe, deren Transport durch Zell- und Organbarrieren mittels der erfindungsgemäßen Verbindungen der Formel (I) verbessert werden kann, seien insbesondere die Folgenden genannt:

Als Antibiotika seien beispielhaft die Fluorchinolone genannt. Fluorchinolone sind unter anderem Verbindungen, wie sie in folgenden Dokumenten offenbart sind: US 4 670 444 (Bayer AG), US 4 472 405 (Riker Labs), US 4 730 000 (Abbott), US 4 861 779 (Pfizer), US 4 382 892 (Daiichi), US 4 704 459 (Toyama), als konkrete Beispiele seien genannt: Benofloxacin, Binfloxacin, Cinoxacin, Ciprofloxacin, Danofloxacin, Difloxacin, Enoxacin, Enrofloxacin, Fleroxacin, Ibafloxacin, Levofloxacin, Lomefloxacin, Marbofloxacin, Moxifloxacin, Norfloxacin, Ofloxacin, Orbifloxacin, Pefloxacin, Pipemidsäure, Temafloxacin, Tosufloxacin, Sarafloxacin, Sparfloxacin.

Eine bevorzugte Gruppe von Fluorchinolonen sind die der Formel (II) oder (III): 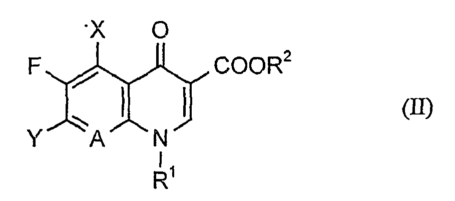 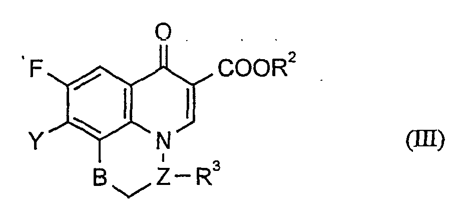 in welchen
- X: für Wasserstoff, Halogen, C₁₋₄-Alkyl, C₁₋₄-Alkoxy, NH₂ steht,
- Y: für Reste der Strukturen 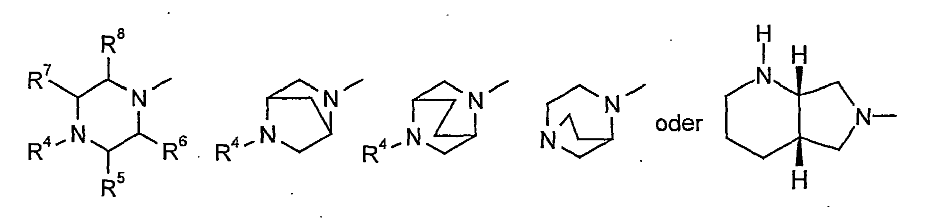 steht, worin
R⁴ für gegebenenfalls durch Hydroxy oder Methoxy substituiertes geradkettiges oder verzweigtes C₁-C₄-Alkyl, Cyclopropyl, Acyl mit 1 bis 3 C-Atomen steht,
R⁵ für Wasserstoff, Methyl, Phenyl, Thienyl oder Pyridyl steht,
R⁶ für Wasserstoff oder C₁₋₄-Alkyl steht,
R⁷ für Wasserstoff oder C₁₋₄-Alkyl steht,
R⁸ für Wasserstoff oder C₁₋₄-Alkyl steht, sowie
R¹ für einen Alkylrest mit 1 bis 3 Kohlenstoffatomen, Cyclopropyl, 2-Fluorethyl, Methoxy, 4-Fluorphenyl, 2,4-Difluorphenyl oder Methylamino steht,
R² für Wasserstoff oder gegebenenfalls durch Methoxy oder 2-Methoxyethoxy substituiertes Alkyl mit 1 bis 6 Kohlenstoffatomen sowie Cyclohexyl, Benzyl, 2-Oxopropyl, Phenacyl, Ethoxycarbonylmethyl, Pivaloyloxymethyl steht,
R³ für Wasserstoff, Methyl oder Ethyl steht und
A für Stickstoff, =CH-, =C(Halogen)-, =C(OCH₃)-, =C(CH₃)- oder =C(CN) steht,
B für Sauerstoff, gegebenenfalls-durch Methyl oder Phenyl substituiertes =NH oder =CH₂ steht,
Z für =CH- oder =N- steht, und deren pharmazeutisch verwendbaren Salze und Hydrate.

Die Verbindungen der Formeln (II) und (III) können in Form ihrer Racemate oder in enantiomeren Formen vorliegen.

Bevorzugt sind Verbindungen der Formel (II),
in welcher
- A: für =CH- oder =C-CN steht,
- R¹: für gegebenenfalls durch Halogen substituiertes C₁-C₃-Alkyl oder Cyclopropyl steht,
- R²: für Wasserstoff oder C₁₋₄-Alkyl steht,
- Y: für Reste der Strukturen 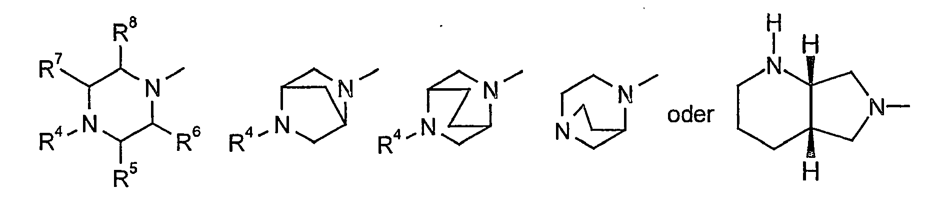 steht, worin
R⁴ für gegebenenfalls durch Hydroxy substituiertes geradkettiges oder verzweigtes C ₁-C₃-Alkyl, Oxalkyl mit 1 bis 4 C-Atomen steht,
R⁵ für Wasserstoff, Methyl oder Phenyl steht,
R⁷ für Wasserstoff oder Methyl steht, und deren pharmazeutisch verwendbaren Hydrate und Salze.

Besonders bevorzugt sind Verbindungen der Formel (II),
in welcher
- A: für =CH- oder =C-CN steht,
- R¹: für Cyclopropyl steht,
- R²: für Wasserstoff, Methyl oder Ethyl steht,
- Y: für Reste der Strukturen 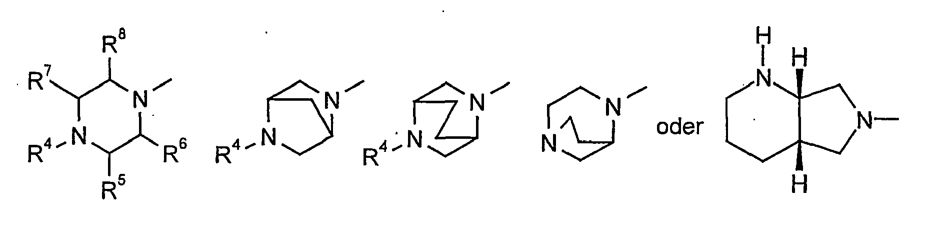 steht, worin
R⁴ für Methyl, gegebenenfalls durch Hydroxy substituiertes Ethyl steht,
R⁵ für Wasserstoff oder Methyl steht,
R⁷ für Wasserstoff oder Methyl steht, und deren pharmazeutisch verwendbaren Salze und Hydrate.

Als Salze kommen pharmazeutisch verwendbare Säureadditionssalze und basische Salze in Frage.

Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen. Ferner lassen sich die erfindungsgemäßen Verbindungen an saure oder basische Ionenaustauscher binden. Als pharmazeutisch verwendbare basische Salze seien die Alkalisalze, beispielsweise die Natrium- oder Kaliumsalze, die Erdalkalisalze, beispielsweise die Magnesium-, oder Calciumsalze; die Zinksalze, die Silbersalze und die Guanidiniumsalze genannt.

Unter Hydraten werden sowohl die Hydrate der Fluorchinolone selbst als auch die Hydrate von deren Salzen verstanden.

Als besonders bevorzugte Fluorchinolone seien die in WO 97/31001 beschriebenen Verbindungen genannt, insbesondere 8-Cyan-1-cyclopropyl-7-((1S,6S)-2,8-diazabicyclo[4.3.0]nonan-8-yl)-6-fluor-1,4-dihydro-4-oxo-3-chinolincarbonsäure (Pradofloxacin) mit der Formel

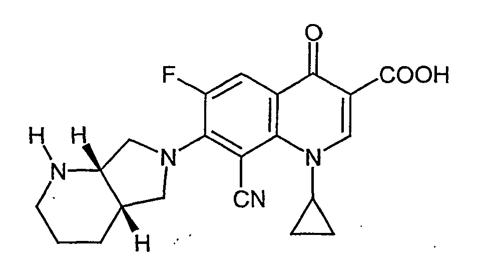

Weiterhin besonders bevorzugt eingesetzt wird Enrofloxacin:
1-Cyclopropyl-7-(4-ethyl-1-piperazinyl)-6-fluor-1,4-dihydro-4-oxo-3-chinolincarbonsäure

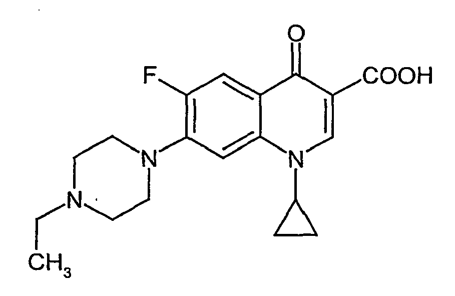

Als Depsipeptide seien insbesondere die cyclischen Depsipeptide genannt. Bevorzugte cyclische Depsipeptide sind solche mit 18 bis 24 Ringatomen, insbesondere mit 24 Ringatomen.

Zu den Depsipeptiden mit 18 Ringatomen zählen Verbindungen der allgemeinen Formel (IV): 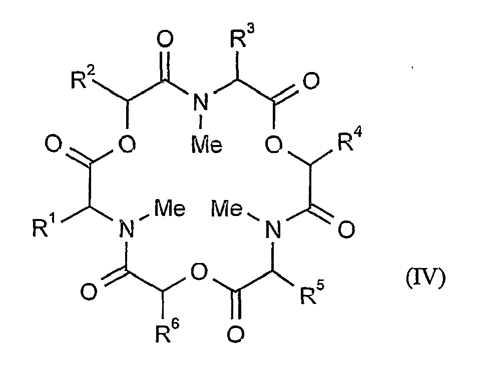 in welcher
- R¹, R³ und R⁵: unabhängig voneinander für Wasserstoff, geradkettiges oder verzweigtes Alkyl mit bis zu 8 Kohlenstoffatomen, Hydroxyalkyl, Alkanoyloxyalkyl, Alkoxyalkyl, Aryloxyalkyl, Mercaptoalkyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Guanidinoalkyl, das gegebenenfalls durch einen oder zwei Benzyloxycarbonylreste oder durch einen, zwei, drei oder vier Alkylreste substituiert sein kann, Alkoxycarbonylaminoalkyl, 9-Fluorenylmethoxycarbonyl(Fmoc)aminoalkyl, Alkenyl, Cycloalkyl, Cycloalkylalkyl sowie gegebenenfalls substituiertes Arylalkyl, wobei als Substituenten genannt seien Halogen, Hydroxy, Alkyl und Alkoxy, stehen,
- R², R⁴ und R⁶: unabhängig voneinander für Wasserstoff, geradkettiges oder verzweigtes Alkyl mit bis zu 8 Kohlenstoffatomen, Hydroxyalkyl, Mercaptoalkyl, Alkanoyloxyalkyl, Alkoxyalkyl, Aryloxyallcyl, Alkylthioalkyl, Alkylsulfinylalkyl, Alkylsulfonylalkyl, Carboxyalkyl, Alkoxycarbonylalkyl, Arylalkoxycarbonylalkyl, Carbamoylalkyl, Aminoalkyl, Alkylaminoalkyl, Dialkylaminoalkyl, Alkoxycarbonylaminoalkyl, Alkenyl, Cycloalkyl, Cycloalkylalkyl gegebenenfalls substituiertes Aryl oder Arylalkyl wobei als Substituenten genannt seien Halogen, Hydroxy, Alkyl, Alkoxy, stehen,
sowie deren optische Isomere und Racemate.

Bevorzugt sind Verbindungen der Formel (IV),
in welcher
- R¹, R³ und R⁵: unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec,-Oktyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxy-ethyl, Mercapto-C₁-C₆-alkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl, C₁-C₄-Alkylsulfonyl-C₁-C₆-alkyl, insbesondere Methylsulfonylethyl, Carboxy-C₁-C₆-alkyl, insbesondere Carboxymethyl, Carboxyethyl, C₁-C₄-Akoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonylmethyl, Carbamoyl-C₁-C₆-alkyl, insbesondere Carbamoylmethyl, Carbamoyl-ethyl, Amino-C₁-C₆-alkyl, insbesondere Aminopropyl, Aminobutyl, C₁-C₄-Akylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, Guanido-C₁-C₆-alkyl, insbesondere Guanidopropyl, C₁-C₄-Alkoxycarbonylamino-C₁-C₆-alkyl, insbesondere tert.-Butoxycarbonylaminopropyl, tert.-Butoxycarbonylaminobutyl, 9-Fluorenylmethoxycarbonyl-(Fmoc)amino-C₁-C₆-alkyl, insbesondere 9-Fluorenyl-methoxycarbonyl(Fmoc)aminopropyl, 9-Fluorenylmethoxycarbonyl(Fmoc)aminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl-C₁-C₄-alkyl, insbesondere Phenylmethyl das gegebenenfalls durch Reste aus der Reihe Halogen, insbesondere Fluor, Chlor Brom oder Iod, Hydroxy, C₁-C₄- Alkoxy, insbesondere Methoxy oder Ethoxy, C₁-C₄-Alkyl, insbesondere Methyl, substituiert sein kann,
- R², R⁴ und R⁶: unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C₁-C₆-alkyl, insbesondere Hydroxymethyl, 1-Hydroxyethyl, C₁-C₄-Alkanoyloxy-C₁-C₆-alkyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxy-ethyl, Mercapto-C₁-C₆-alkyl, insbesondere Mercaptomethyl, C₁-C₄-Alkylthio-C₁-C₆-alkyl, insbesondere Methylthioethyl, C₁-C₄-Alkylsulfinyl-C₁-C₆-alkyl, insbesondere Methylsulfinylethyl, C₁-C₄-Alkylsulfonyl-C₁-C₆-alkyl, insbesondere Methylsulfonylethyl, Carboxy-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Arylalkoxycarbonyl-C₁-C₆-alkyl, insbesondere Benzyloxycarbonylmethyl, Carbamoyl-C₁-C₆-alkyl, insbesondere Carbamoylmethyl, Carbamoylethyl, Amino-C₁-C₆-alkyl, insbesondere Aminopropyl, Aminobutyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl, Phenyl-C₁-C₄-alkyl, insbesondere Phenylmethyl das gegebenenfalls durch Reste aus der Reihe Halogen, insbesondere Fluor, Chlor Brom oder Iod, Hydroxy, C₁-C₄- Alkoxy, insbesondere Methoxy oder Ethoxy, C₁-C₄-Alkyl, insbesondere Methyl, substituiert sein kann,
sowie deren optische Isomere und Racemate.

Besonders bevorzugt sind Verbindungen der Formel (IV),
in welcher
- R¹, R³ und R⁵: unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, Hydroxy-C₁-C₆-alkyl, insbesondere Hydroxymethyl, 1-Hydroxyethyl, C₁-C₄-Alkanoyloxy-C₁-C₆-allcyl, insbesondere Acetoxymethyl, 1-Acetoxyethyl, C₁-C₄-Alkoxy-C₁-C₆-alkyl, insbesondere Methoxymethyl, 1-Methoxyethyl, Aryl-C₁-C₄-alkyloxy-C₁-C₆-alkyl, insbesondere Benzyloxymethyl, 1-Benzyloxyethyl, C₁-C₄-Alkoxycarbonylamino-C₁-C₆-alkyl, insbesondere tert.-Butoxycarbonylaminopropyl, tert.-Butoxycarbonylaminobutyl, C₂-C₈-Alkenyl, insbesondere Vinyl, Allyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl-C₁-C₄-alkyl, insbesondere Phenylmethyl das gegebenenfalls durch einen oder mehrere gleiche oder verschiedene der oben angegebenen Reste substituiert sein kann,
- R², R⁴ und R⁶: unabhängig voneinander für geradkettiges oder verzweigtes C₁-C₈-Alkyl, insbesondere Methyl, Ethyl, Propyl, Isopropyl, Butyl, Isobutyl, sec.-Butyl, tert.-Butyl, Pentyl, Isopentyl, sec.-Pentyl, Hexyl, Isohexyl, sec.-Hexyl, Heptyl, Isoheptyl, sec.-Heptyl, tert.-Heptyl, Oktyl, Isooktyl, sec.-Oktyl, Hydroxy-C₁-C₆-alkyl, insbesondere Hydroxymethyl, Aryl-C₁-C₄-alkyloxy-C₁-C₆-alkyl, insbesondere Benzyloxymethyl, 1-Benzyloxyethyl, Carboxy-C₁-C₆-alkyl, insbesondere Carboxymethyl, Carboxyethyl, C₁-C₄-Alkoxycarbonyl-C₁-C₆-alkyl, insbesondere Methoxycarbonylmethyl, Ethoxycarbonylethyl, C₁-C₄-Aryl-alkoxycarbonyl-C₁-C₆-alkyl, insbesondere Benzyloxycarbonylinethyl, C₁-C₄-Alkylamino-C₁-C₆-alkyl, insbesondere Methylaminopropyl, Methylaminobutyl, C₁-C₄-Dialkylamino-C₁-C₆-alkyl, insbesondere Dimethylaminopropyl, Dimethylaminobutyl, C₂-C₈-Alkenyl, insbesondere Vinyl, Allyl, Butenyl, C₃-C₇-Cycloalkyl, insbesondere Cyclopentyl, Cyclohexyl, Cycloheptyl, C₃-C₇-Cycloalkyl-C₁-C₄-alkyl, insbesondere Cyclopentylmethyl, Cyclohexylmethyl, Cycloheptylmethyl, Phenyl, Phenyl-C₁-C₄-alkyl, insbesondere Phenylmethyl das gegebenenfalls durch einen oder mehrere gleiche oder verschiedene der oben angegebenen Reste substituiert sein kann,
sowie deren optische Isomere und Racemate.

Ganz besonders bevorzugt sind Verbindungen der Formel (IV),
in welcher
- R¹, R³ und R⁵: unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl C₂-C₈-Alkenyl, insbesondere Allyl, C₃-C₇-Cycloalkyl-C₁-C₄-alkyl, insbesondere Cyclohexylmethyl, Phenyl-C₁-C₄-alkyl, insbesondere Phenylmethyl,
- R², R⁴ und R⁶: unabhängig voneinander für geradkettiges oder verzweigtes C₁-C₈-Alkyl, insbesondere Methyl, Ethyl, Propyl, Isopropyl, Butyl, Isobutyl, sec.-Butyl, Pentyl, Isopentyl, sec.-Pentyl, Hexyl, Isohexyl, sec.-Hexyl, Heptyl, Isoheptyl, sec.-Heptyl, Oktyl, Isooktyl, sec.-Oktyl, C₂-C₈- Alkenyl, insbesondere Vinyl, Allyl, C₃-C₇-Cycloalkyl-C₁-C₄-alkyl, insbesondere Cyclohexylmethyl, Phenyl-C₁-C₄-alkyl, insbesondere Phenylmethyl das gegebenenfalls durch einen oder mehrere gleiche oder verschiedene der oben angegebenen Reste substituiert sein kann,
sowie deren optische Isomere und Racemate.

Im Sinne der vorliegenden Erfindung können alle Verbindungen der allgemeinen Formel (IV), die in optisch aktiven, stereoisomeren Formen oder als racemische Gemische vorliegen können, verwendet werden. Vorzugsweise werden jedoch die optisch aktiven, stereoisomeren Formen der Verbindungen der allgemeinen Formel (IV) erfindungsgemäß verwendet.

Im Einzelnen seien folgende Verbindungen der allgemeinen Formel (IV) genannt, in welcher
die Reste R¹ bis R⁶ die folgende Bedeutung haben:

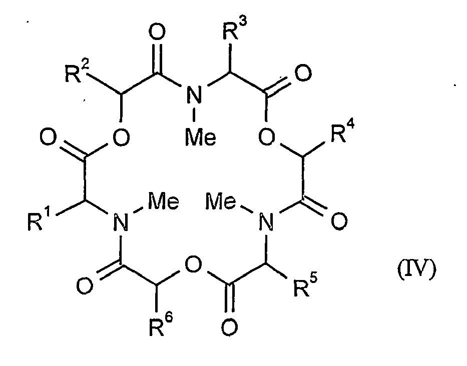

| **R¹** | **R²** | **R³** | **R⁴** | **R⁵** | **R⁶** |
|---|---|---|---|---|---|
| -CHMeCH₂Me | -Cyclohexyl | -CHMeCH₂Me | -Me | -CHMeCH₂Me | -Me |
| -CHMeCH₂Me | -Cyclohexyl | -CHMeCH₂Me | -Me | -CHMeCH₂Me | -Cyclohexyl |
| -CHMeCH₂Me | -CH₂-Phe | -CHMeCH₂Me | -Me | -CHMeCH₂Me | -Me |
| -CHMeCH₂Me | -CH₂-Phe | -CHMeCH₂Me | -Me | -CHMeCH₂Me | -CH₂-Phe |
| -CHMeCH₂Me | -(CH₂)₃-Me | -CHMeCH₂Me | -Me | -CHMeCH₂Me | -Me |
| -CHMeCH₂Me | -(CH₂)₃-Me | -CHMeCH₂Me | -Me | -CHMeCH₂Me | -(CH₂)₃-Me |
| -CHMe₂ | -CH₂-Phe | -CHMeCH₂Me | -Me | -CHMeCH₂Me | -Me |
| -CH₂-Phe | -CHMe₂ | -CH₂-Phe | -CHMe₂ | -CHMeCH₂Me | -CHMe₂ |
| -CH₂CHMe₂ | -CH₂-Phe | -CH₂CHMe₂ | -Me | -CH₂CHMe₂ | -CH₂-Phe |
| -(CH₂)₃-Me | -Me | -CHMeCH₂Me | -Me | -CHMeCH₂Me | -Me |
| -CHMe₂ | -Me | -CHMe₂ | -Me | -CHMe₂ | -Me |
| -CH₂-Me | -Me | -CH₂-Me | -Me | -CH₂-Me | -Me |
| -(CH₂)₂-Me | -Me | -(CH₂)₂-Me | -Me | -(CH₂)₂-Me | -Me |
| -(CH₂)₃-Me | -Me | -(CH₂)₃-Me | -Me | -(CH₂)₃-Me | -Me |
| -CH₂-CH=CH₂ | -Me | -CH₂-CH=CH₂ | -Me | -(CH₂)-CH=CH₂ | -Me |
| -CHMeCH₂Me | -Me | -CHMeCH₂Me | -Me | -CHMeCH₂Me | -CH₂-Me |
| -CHMeCH₂Me | -Me | -CHMeCH₂Me | -Me | -CHMeCH₂Me | -(CH₂)₂-Me |
| -CHMeCH₂Me | -Me | -CHMeCH₂Me | -Me | -CHMeCH₂Me | -(CH₂)₃-Me |
| -CHMeCH₂Me | -Me | -CHMeCH₂Me | -Me | -CH₂Me | -Me |
| -CHMeCH₂Me | -Me | -CHMeCH₂Me | -Me | -(CH₂)₂-Me | -Me |
| -Cyclohexyl | -Me | -Cyclohexyl | -Me | -Cyclohexyl | -Me |
| -CH₂CHMe₂ | -Cyclohexyl | -CH₂CEMe₂ | -Me | -CH₂CHMe₂ | -Cyclohexyl |
| -CH₂CHMe₂ | -Cyclohexyl | -CH₂CHMe₂ | -Me | -CH₂CHMe₂ | -Me |
| -CHMeCH₂Me | -CHMe₂ | -CHMeCH₂Me | -CHMe₂ | -CHMeCH₂Me | -Me |
| -CH₂-Phe | -Me | -CH₂-Phe | -Me | -CH₂-Phe | -Me |
| -Cyclohexyl | -Me | -Cyclohexyl | -Me | -Cyclohexyl | -Me |
| -CHMe₂ | -CHMe₂ | -CHMe | -Me | -CHMe₂ | -Me |
| -CHMe₂ | -CHMe₂ | -CHMe₂ | -CHMe₂ | -CHMe₂ | -Me |
| -CH₂-Me | -CHMe₂ | -CH₂Me | -Me | -CH₂-Me | -Me |
| -CH₂-Me | -CHMe₂ | -CHMe₂ | -CHMe₂ | -CH₂-Me | -Me |
| -(CH₂)₂-Me | -CHMe₂ | -(CH₂)₂-Me | -Me | -(CH₂)₂-Me | -Me |
| -(CH₂)₂-Me | -CHMe₂ | -(CH₂)₂-Me | -CHMe₂ | -(CH₂)₂-Me | -Me |
| -(CH₂)₃-Me | -CHMe₂ | -(CH₂)₃-Me | -Me | -(CH₂)₃-Me | -Me |
| -(CH₂)₃-Me | -CHMe₂ | -(CH₂)₃-Me | -CHMe₂ | -(CH₂)₃-Me | -Me |
| -CH₂-CH=CH₂ | -CHMe₂ | -CH₂-CH=CH₂ | -Me | -CH₂-CH=CH₂ | -Me |
| -CH₂-CH=CH₂ | -CHMe₂ | -CH₂-CH=CH₂ | -CHMe₂ | -CH₂-CH=CH₂ | -Me |
| -Me | -Me | -CHMeCH₂Me | -Me | -CH₂-Me | -Me |
| -Me | -Me | -CHMeCH₂Me | -Me | -(CH₂)₃-Me | -Me |

| | | | | | |
|---|---|---|---|---|---|
| Me = Methyl; Phe = Phenyl | | | | | |

Weiterhin sei als Depsipeptid die aus EP-OS 382 173 bekannte Verbindung PF 1022 der folgenden Formel genannt:

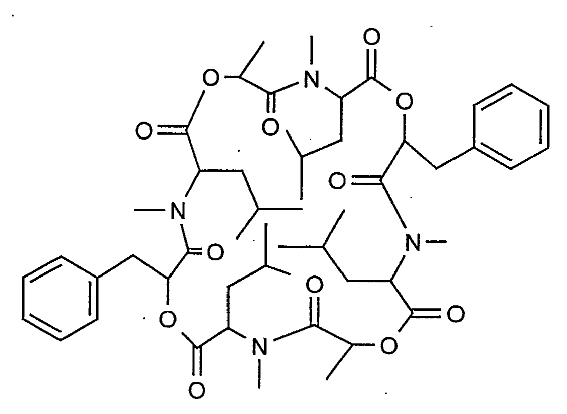

Außerdem seien als Depsipeptide die aus der PCT-Anmeldung WO 93/19053 bekann-ten Verbindungen genannt.

Insbesondere seien aus PCT-Anmeldung WO 93/19053 die Verbindungen der folgenden Formel genannt: 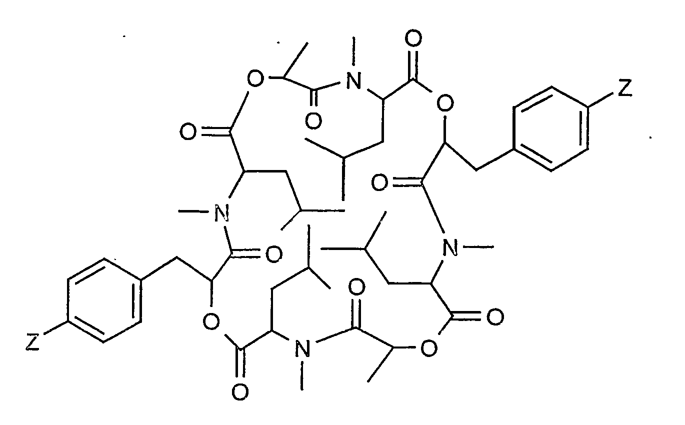 in welcher
- Z: für N-Morpholinyl, Amino, Mono- oder Dimethylamino steht.

Von diesen besonders bevorzugt ist Emodepsid, die Verbindung der vorstehenden Formel, in der beide Reste Z für den N-Morpholinylrest stehen.

Außerdem seien Verbindungen der folgenden Formel genannt: 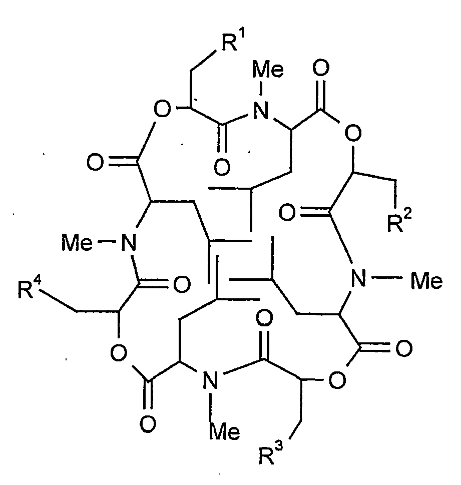 in welcher
- R¹, R², R³, R⁴: unabhängig voneinander für Wasserstoff, C₁-C₁₀-Alkyl oder Aryl, insbesondere Phenyl stehen, die gegebenenfalls substituiert sind durch Hydroxy, C₁-C₁₀-Alkoxy oder Halogen.

Die Verbindungen der allgemeinen Formel (IV) sind bekannt und können nach den in EP-A-382 173, DE-A 4 317 432, DE-A 4 317 457, DE-A 4 317 458, EP-A-634 408, EP-A-718 293, EP-A-872 481, EP-A-685 469, EP-A-626 375, EP-A-664 297, EP-A-669 343, EP-A-787 141, EP-A-865 498, EP-A-903 347 beschriebenen Verfahren erhalten werden.

Zu den cyclischen Depsipeptiden mit 24 Ringatomen zählen auch Verbindungen der allgemeinen Formel (Na) 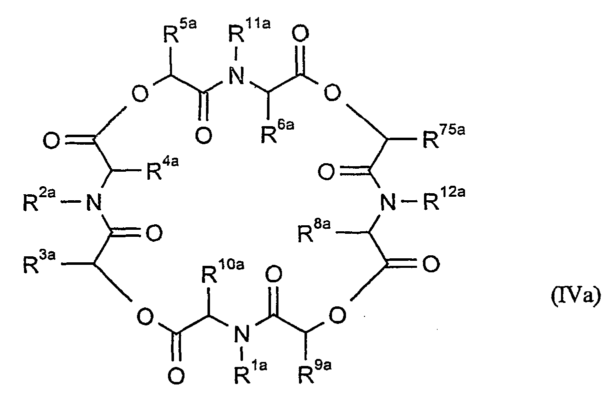 in welcher
- R^{1a}, R^{2a}, R^{11a} und R^{12a}: unabhängig voneinander für C₁₋₈-Alkyl, C₁₋₈-Halogenalkyl, C₃₋₆-Cycloalkyl, Aralkyl, Aryl stehen,
- R^{3a}, R^{5a}, R^{7a}, R^{9a}: unabhängig voneinander für Wasserstoff oder geradkettiges oder verzweigtes C₁₋₈-Alkyl steht, das gegebenenfalls durch Hydroxy, C₁₋₄-Alkoxy, Carboxy, 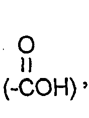 Carboxamid, 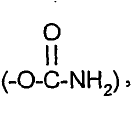 Imidazolyl, Indolyl, Guanidino, -SH oder C₁₋₄-Alkylthio substituiert sein kann und ferner für Aryl oder Aralkyl die durch Halogen, Hydroxy, C₁₋₄-Alkyl, C₁₋₄-Alkoxy substituiert sein können, steht,
- R^{4a}, R^{6a}, R^{8a}, R^{10a}: unabhängig voneinander für Wasserstoff, geradkettiges C₁₋₅-Alkyl, C₂₋₆-Alkenyl, C ₃₋₇-Cycloalkyl, die gegebenenfalls durch Hydroxy, C₁₋₄-Alkoxy, Carboxy, Carboxamid, Imidazolyl, Indolyl, Guanidino, SH oder C₁₋₄-Alkylthio substituiert sein können, sowie für Aryl oder Aralkyl die durch Halogen, Hydroxy, C₁₋₄-Alkyl, C₁₋₄-Alkoxy substituiert sein können, stehen
sowie deren optische Isomere und Racemate.

Bevorzugt werden Verbindungen der Formel (IVa) eingesetzt, in welcher
- R^{1a}, R^{2a}, R^{11a} und R^{12a}: unabhängig voneinander für Methyl, Ethyl, Propyl, Isopropyl, n-, s-, t-Butyl oder Phenyl, das gegebenenfalls substituiert ist durch Halogen, C₁₋₄-Alkyl, OH, C₁₋₄-Alkoxy, sowie für Benzyl oder Phenylethyl stehen, die gegebenenfalls durch die bei Phenyl angegebenen Reste substituiert sein können;
- R^{3a} bis R^{10a}: die oben angegebene Bedeutung haben.

Besonders bevorzugt sind Verbindungen der Formel (Ia), in welcher
- R^{1a}, R^{2a}, R^{11a} und -R^{12a}: unabhängig voneinander für Methyl, Ethyl, Propyl, Isopropyl oder n-, s-, t-Butyl stehen,
- R^{3a}, R^{5a}, R^{7a}, R^{9a}: für Wasserstoff, geradkettiges oder verzweigtes C₁₋₈-Alkyl, insbesondere Methyl, Ethyl, Propyl, i-Propyl, n-, s-, t-Butyl, die gegebenenfalls durch C₁₋₄-Alkoxy, insbesondere Methoxy, Ethoxy, Imidazolyl, Indolyl oder C₁₋₄-Alkylthio, insbesondere Methylthio, Ethylthio substituiert sein können, ferner für Phenyl, Benzyl oder Phenethyl stehen, die gegebenenfalls durch Halogen insbesondere Chlor substituiert sein können.
- R^{4a}, R^{6a}, ^{8a}, R^{10a}: R unabhängig voneinander für Wasserstoff, Methyl, Ethyl, n-Propyl, n-Butyl, Vinyl, Cyclohexyl, die gegebenenfalls durch Methoxy, Ethoxy, Imidazolyl, Indolyl, Methylthio, Ethylthio substituiert sein können sowie für Isopropyl, s-Butyl ferner für gegebenenfalls halogensubstituiertes Phenyl, Benzyl oder Phenylethyl stehen.

Die Verbindungen der Formel (IVa) können ebenfalls nach den in EP-A-382 173, DE-A 4 317 432, DE-A 4 317 457, DE-A 4 317 458, EP-A-634 408, EP-A-718 293, EP-A-872 481, EP-A-685 469, EP-A-626 375, EP-A-664 297, EP-A-669 343, EP-A-787 141, EP-A-865 498, EP-A-903 347 beschriebenen Verfahren erhalten werden.

Als makrocyclische Lactone seien Avermectine, 22,23-Dihydroavermectine B₁ (Ivermectine) und Milbemycine genannt.

Als Avermectine und deren Derivate seien genannt Stoffe und Stoffgemische von makroliden Lactonen der allgemeinen Formel (V) 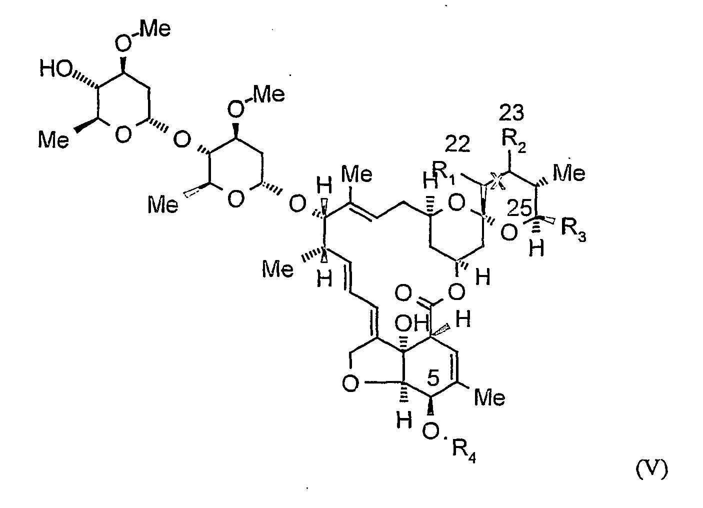 in welcher
die Reste R¹ bis R⁴ die in der nachfolgenden Tabelle 1 angegebene Bedeutung haben und X für eine Einfach- oder Doppelbindung zwischen der C₂₂- und C₂₃-Position (-C₂₂R¹-X-C₂₃R²-) stehen kann.

Im Falle einer Doppelbindung befinden sich keine Substituenten (R¹, R²) an der C₂₂- und C₂₃-Position.

**Tabelle 1**

| **Makrocyclisches Lacton** | **-C₂₂R¹-X-C₂₃R²-** | **R³** | **R⁴** |
|---|---|---|---|
| Avermectin A₁ₐ | -CH=CH- | -sec-Bu | -Me |
| Avermectin A_{1b} | -CH=CH- | -iso-Pr | -Me |
| Avermectin A₂ₐ | -CH₂-CHOH- | -sec-Bu | -Me |
| Avermectin A_{2b} | -CH₂-CHOH- | -iso-Pr | -Me |
| Avermectin B₁ₐ | -CH=CH- | -sec-Bu | -H |
| Avermectin B_{1b} | -CH=CH- | -iso-Pr | -H |
| Avermectin B₂ₐ | -CH₂-CHOH- | -sec-Bu | -H |
| Avermectin B_{2b} | -CH₂-CHOH- | -iso-Pr | -H |
| 22,23-Dihydroavermectin B₁ₐ | -CH₂-CH₂- | -sec-Bu | -H |
| 22,23-Dihydroavermectin B_{1b} | -CH₂-CH₂- | -iso-Pr | -H |
| Doramectin | -CH=CH- | -Chx | -H |

| | | | |
|---|---|---|---|
| 22,23-Dihydroavermectin B₁ steht für Ivermectin B₁; sec-Bu = sekundär Butyl; iso-Pr = Isopropyl; Chx = Cyclohexyl; -Me = Methyl | | | |

Die Avermectine und 22,23-Dihydroavermectine B₁ (Ivermectine) der allgemeinen Formel (I) werden in der Regel als Gemische eingesetzt. Von besonderem Interesse ist hierbei das Produkt Abamectin, das im wesentlichen die Avermectine B₁ enthält, und deren Hydrierungsprodukte die 22,23-Dihydroavermectine B₁ (Ivermectin).

Die mit "b" bezeichneten Verbindungen der makrocyclischen Lactone, die in der C₂₅-Position einen iso-Propylrest besitzen, müssen nicht notwendigerweise von den "a" Verbindungen, welche eine sec-Butylgruppe in der C₂₅-Position haben, getrennt werden. Es wird generell das Gemisch beider Substanzen, bestehend aus > 80 % sec-Butylderivat (B₁ₐ) und < 20 % iso-Propylderivat (B_{1b}) isoliert, und kann erfindungsgemäß verwendet werden. Zudem können bei den Stereoisomeren die Substituenten in der C₁₃- und C₂₃-Position sowohl α- als auch β-ständig am Ringsystem angeordnet sein, d. h. sich oberhalb oder unterhalb der Molekülebene befinden.

Die Milbemycine haben die gleiche makrolide Ringstruktur wie Avermectine oder 22,23-Dihydroavermectine B₁ (Ivermectine), tragen aber keinen Substituenten (d.h. fehlendes Oleandrose-Disaccharidfragment) in Position 13 (R⁵ = Wasserstoff).

Beispielhaft seien als Milbemycine aus der Klasse der macrocyclischen Lactone die Verbindungen mit der allgemeinen Formel (VI) genannt 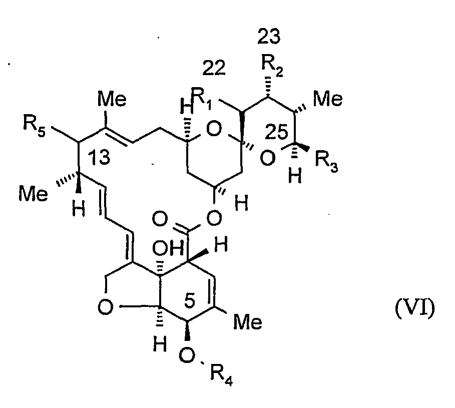 in welcher
die Reste R¹ bis R⁵ die in der nachfolgenden Tabelle 2 angegebene Bedeutung haben:

**Tabelle 2**

| **Makrocyclisches Lacton** | **R¹** | **R²** | **R³** | **R⁴** | **R⁵** |
|---|---|---|---|---|---|
| Milbemycin B41 D | -H | -H | -iso-Pr | -H | -H |
| Nemadectin | -H | -OH | 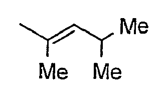 | -H | -H |
| Moxidectin | -H | N-O-Me | 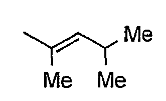 | -H | -H |

| | | | | | |
|---|---|---|---|---|---|
| iso-Pr = Isopropyl | | | | | |

Besonders bevorzugt sind: Avermectin B₁ₐ/B_{1b}; 22,23-Diliydroavermectin B₁ₐ/B_{1b} bzw. Ivermectin B₁ₐ/B_{1b}); Doramectin; Moxidectin.

Die Verbindungen der Formel (I) eignen zur Verwendung bei Menschen und Tieren, und zwar in der Tierhaltung und Tierzucht bei Nutz-, Zucht-, Zoo-, Labör-, Versuchs- und Hobbytieren (Heimtiere).

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere; Pelztiere wie z.B. Nerze, Chinchilla, Waschbär; Vögel wie z.B. Hühner, Gänse, Puten, Enten, Tauben, Strauße, Vogelarten für Heim- und Zoohaltung. Ferner gehören Nutz- und Zierfische zu den möglichen Zielgruppen.

Zu Labor- und Versuchstieren gehören Mäuse, Ratten, Gerbils, Meerschweinchen, Goldhamster, Hunde und Katzen.

Zu den Hobbytieren gehören Kaninchen, Hasen, Reptilien, Amphibien sowie bevorzugt Hunde und Katzen.

Zu den Fischen gehören Nutz-, Zucht-, Aquarien- und Zierfische aller Altersstufen, die in Süß- und Salzwasser leben. Zu den Nutz- und Zuchtfischen zählen z.B. Karpfen (z. B. Koi-Karpfen), Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabream (Pagurus major), Seabass (Dicentrarchus labrax), Grey mullet (Mugilus cephalus), Pompano, Gilthead seabream (Sparus auratus), Tilapia spp., Cichliden-Arten wie z.B. Plagioscion, Channel catfish etc.

Die Anwendung kann sowohl prophylaktisch als auch therapeutisch erfolgen.

Die Anwendung erfolgt in Form von geeigneten Zubereitungen, und zwar üblicherweise oral in Form von Pulver, Zäpfchen, Tabletten, Kapseln, Pasten, Tränken, Granulaten, Drenchen, Boli, medikiertem Futter oder Trinkwasser.

Geeignete Zubereitungen sind beispielsweise:
Orale Lösungen, Konzentrate zur oralen Verabreichung nach Verdünnung, Gele;
Emulsionen und Suspension zur oralen Anwendung; Halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln, Aerosole und Inhalate, wirkstoffhaltige Formkörper.

Injektionslösungen werden hergestellt, indem gegebenenfalls Zusätze wie Lösungsvermittler, Säuren, Basen, Puffersalze, Antioxidantien und Konservierungsmittel und der Wirkstoff oder die Wirkstoffe einem geeigneten Lösungsmittel oder Lösungsmittelgemisch zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Kohlenwasserstoffe, Propylenglykol, Polyethylenglykole, N-Methylpyrrolidon, Glycerinformal, Solketal, 2-Pyrrolidon, Dimethylacetamid, Glycofurol (Tetraglykol), Benzylbenzoat sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Stoffe, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester und Poloxamere.

Konservierungsmittel sind: Benzylalkohol, Ethanol, n-Butanol, m-Kresol, Trichlorbutanol, Butylhydroxyanisol (BHA), Butylhydroxytoluol (BHT), Benzalkoniumchlorid.

Orale Lösungen werden direkt angewendet. Konzentrate werden nach vorheriger Verdünnung auf die Anwendungskonzentration oral angewendet. Orale Lösungen und Konzentrate werden, wie oben bei den Injektionslösungen beschrieben, hergestellt, wobei auf steriles Arbeiten verzichtet werden kann.

Für orale Lösungen eignen sich folgende Konservierungsmittel: p-Hydroxybenzoesäureester, Butylhydroxyanisol (BHA), Butylhydroxytoluol (BHT) sowie Salze der Sorbinsäure, Propionsäure, Milchsäure und Benzoesäure.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate und Xanthan.

Emulsionen können oral angewendet werden.

Emulsionen sind entweder vom Typ Wasser in Öl oder von Typ Öl in Wasser.

Sie werden hergestellt, indem man den Wirkstoff entweder in der hydrophoben oder in der hydrophilen Phase dispergiert und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Erdnußöl, Sojaöl, Rizinusöl, Baumwollsamenöl, synthetische Triglyceride wie Capryl/Caprinsäure-triglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter, eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylenglykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt:
nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt:
Viskositätserhöhende und die Emulsion stabilisierende Stoffe wie Carboxymethylcellulose, Methylcellulose und andere Cellulose- und Stärke-Derivate, Polyacrylate, Poloxamere, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien, Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe. Antioxidantien sind Sulfite oder Metabisulfite wie Kaliummetabisulfit, Ascorbinsäure, Butylhydroxytoluol, Butylhydroxyanisol, Tocopherol, Cysteamin, Gallussäuresalze, wie z. B. Propylgallat oder Aminosäuren wie z. B. Cystein.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Verbindungen der Formel (I) werden als Kombinationspräparat mit einem oder mehreren pharmazeutischen Wirkstoffen verabreicht, wobei die Anwendung von Verbindung der Formel (I) und Wirkstoff gleichzeitig, getrennt oder zeitlich abgestuft erfolgen kann. Bei der gleichzeitigen Anwendung können Wirkstoff(e) und Verbindung der Formel (I) in einer gemeinsamen galenischen Formulierung vorliegen.

Anwendungsfertige Zubereitungen enthalten die Verbindungen der Formel (I) in Konzentrationen von 10 ppm bis 20 Gewichtsprozent, bevorzugt von 0,01 bis 10 Gewichtsprozent.

Zubereitungen die vor Anwendung verdünnt werden, enthalten die Verbindungen der Formel (I) in Konzentrationen von 0,5 bis 90 Gewichtsprozent, bevorzugt von 1 bis 50 Gewichtsprozent.

### Formulierbeispiele

Die Substanzen werden gemischt, gegebenenfalls sterilfiltriert, in geeignete Behältnisse überführt, gegebenenfalls autoklaviert und verschlossen.

### Beispiel 1

### Lösung

| | |
|---|---|
| 0,75 g | Mebendazol |
| 3,75 g | Verbindungen der Formeln (A) und (B) im Verhältnis 9:1 |
| ad 100 g | N-Methylpyrrolidon |

### Beispiel 2

### Suspension

| | |
|---|---|
| 1,0 g | Mebendazol |
| 5,0 g | Verbindungen der Formeln (A) und (B) im Verhältnis 9:1 |
| 50 g | N-Methylpyrrolidon |
| ad 100 g | Sesamöl |

### Beispiel 3

### Suspension

| | |
|---|---|
| 1,0 g | Mebendazol |
| 5,0 g | Verbindungen der Formeln (A) und (B) im Verhältnis 9:1 |
| 70 g | Glycerinformal |
| ad 100 g | Aqua demin. |

### Beispiel 4

### Suspension

| | |
|---|---|
| 1,0 g | Mebendazol |
| 1,0 g | Tween 80 |
| 5,0 g | Verbindungen der Formeln (A) und (B) im Verhältnis 9:1 |
| ad 100 g | Miglyol812 |

### Beispiel 5

| | |
|---|---|
| 0,72 g | Mebendazol |
| 1,13 g | Verbindungen der Formeln (A) und (B) im Verhältnis 9:1 |

werden auf 108 ml aufgefüllt mit einer wäßrigen Lösung, enthaltend 10 Vol.-% Ethanol.

### Beispiel 6

| | |
|---|---|
| 0,72 g | Mebendazol |
| 1,13 g | Verbindungen der Formeln (A) und (B) im Verhältnis 9:1 |

werden auf 108 ml aufgefüllt mit einer wäßrigen Lösung, enthaltend 10 Vol.-% Ethanol und 20 Vol.-% Cremophor EL.

### Biologische Beispiele

Die Tabelle 3 zeigt die Ergebnisse des Nachweises der Wurmstadien bei experimentell mit Angiostrongylus cantonensis infizierten Wistar-Ratten nach oraler Behandlung mit einer wäßrigen Mebendazol-Suspension (10 mg/kg KGW) und Mebendazol (0.33 mg/kg KGW) in verschiedenen Carrier-Systemen in % im Vergleich zu den Infektionskontrollen und Carrierkontrolle.

**Tabelle 3**

| **Versuchstyp** | **Wurmnachweis im Gehirn** |
|---|---|
| Infektionskontrolle | 55 Würmer nachgewiesen, entspricht 100% |
| Mebendazol wässr. Suspension, 10 mg/kg KGW | 98.2 % |
| Enhancer* alleine | 100 % |
| Enhancer* + Cremophor | 100 % |
| Mebendazol (0.33 mg/kg KGW) + Enhancer* (Lösung gemäß Bsp. 1) | 4 % |
| Mebendazol (0.33 mg/kg KGW) +Enhancer* (Lösung gemäß Beispiel 6) | 6 % |

| | |
|---|---|
| *: Enhancer: Verbindungen der Formeln (A) und (B) im Verhältnis 9:1 | |

Jede Ratte wurde mit exakt 60 L3-Larven infiziert. Ab Tag 5 nach der Infektion wurde 1 x täglich an 3 aufeinanderfolgenden Tagen Mebendazol in den angegebenen Dosierungen und Formulierungen appliziert. Der Wurmnachweis erfolgte am Tag 21 nach Infektion.

Mit Hilfe des Carrier-Systems wird das Medikament Mebendazol durch die Blut-Hirnschranke transportiert. Dadurch kommt es zu einer signifikanten Wurmreduktion im Gehirn bei einer mehr als 30-fach geringeren Mebendazoldosis.

## Patentansprüche

1. Verwendung von Verbindungen der Formel (I) 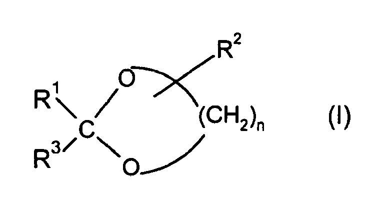 worin
R¹ für einen gegebenenfalls mit einem oder mehreren Halogenatomen substituierten Alkyl-, Alkenyl- oder Alkinylrest mit 2 bis 30 Kohlenstoffatomen steht, wobei gegebenenfalls ein oder mehrere geeignete, nicht benachbarte Kohlenstoff-Kettenglieder durch Sauerstoffatome ersetzt sein können,
R² für Wasserstoff, Hydroxyl, -NH₂, -NR⁴R⁵, -N⁺(R⁴R⁵R⁶), -PR⁷R⁸, -O-P(R⁷R⁸), - P(O)R⁷R⁸, -P⁺(R⁷R⁸R⁹) oder einen C₁₋₅-Alkylrest steht, der gegebenenfalls durch Hydroxyl, C₁₋₄-Alkoxy, -NH₂, Mono- oder Di-C₁₋₄-alkylamino oder einen 5- bis 7-gliedrigen Heterocyclus mit bis zu drei Heteroatomen, ausgewählt aus O, N und S, substituiert ist,
R³ für Wasserstoff steht oder die oben angegebenen Bedeutungen von R¹ annehmen kann,
R⁴, R⁵ und R⁶ unabhängig von einander für Wasserstoff oder C₁₋₅-Alkyl stehen oder zwei der Reste gemeinsam mit dem Stickstoffatom, an das sie gebunden sind, einen 5- bis 7-gliedrigen Heterocyclus bilden, der gegebenenfalls noch ein oder zwei weitere Heteroatome, ausgewählt aus O, N und S, enthalten kann,
R⁷, R⁸ und R⁹ unabhängig von einander für C₁₋₅-Alkyl, C₁₋₅-Alkoxy oder C₆₋₁₂-Aryl stehen
oder
zwei der Reste gemeinsam mit dem Phosphoratom, an das sie gebunden sind, einen 5-7-gliedrigen Heterocyclus bilden, der gegebenenfalls noch ein oder zwei weitere Heteroatome, ausgewählt aus O, N und S, enthalten kann,
n 2, 3 oder 4 bedeutet,
zur Herstellung von Arzneimitteln mit verbessertem Durchtritt eines pharmazeutischen Wirkstoffs durch Zell- und Organbarrieren, wobei das Arzneimittel oral verabreicht wird.

2. Verwendung gemäß Anspruch 1, wobei in der Formel (I)
R¹ für einen gegebenenfalls durch ein oder mehrere Fluor- oder Chloratome substituierten n-Alkylrest mit 2 bis 20 Kohlenstoffatomen, in dem eines der Kohlenstoff-Kettenglieder durch ein Sauerstoffatom ersetzt sein kann, steht.

3. Verwendung gemäß Anspruch 1 oder 2, wobei in der Formel (I)
R¹ besonders bevorzugt für einen n-Alkylrest mit 5 bis 12 Kohlenstoffatomen steht.

4. Verwendung von Verbindungen der Formel (I) 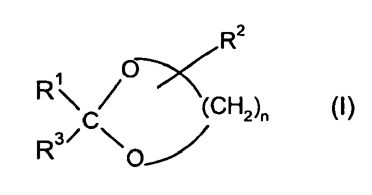 worin
R¹ für einen gegebenenfalls mit einem oder mehreren Halogenatomen substituierten Alkyl-, Alkenyl- oder Alkinylrest mit 2 bis 30 Kohlenstoffatomen steht, wobei gegebenenfalls ein oder mehrere geeignete, nicht benachbarte Kohlenstoff-Kettenglieder durch Sauerstoffatome ersetzt sein können,
R² für Wasserstoff, Hydroxyl, -NH₂, -NR⁴R⁵, -N⁺(R⁴R⁵R⁶), -PR⁷R⁸, -O-P(R⁷R⁸), - P(O)R⁷R⁸, -P⁺(R⁷R⁸R⁹) oder einen C₁₋₅-Alkylrest steht, der gegebenenfalls durch Hydroxyl, C₁₋₄-Alkoxy, -NH₂, Mono- oder Di-C₁₋₄-alkylamino oder einen 5- bis 7-gliedrigen Heterocyclus mit bis zu drei Heteroatomen, ausgewählt aus O, N und S, substituiert ist,
R³ für Wasserstoff steht oder die oben angegebenen Bedeutungen von R¹ annehmen kann,
R⁴, R⁵ und R⁶ unabhängig von einander für Wasserstoff oder C₁₋₅-Alkyl stehen oder zwei der Reste gemeinsam mit dem Stickstoffatom, an das sie gebunden sind, einen 5- bis 7-gliedrigen Heterocyclus bilden, der gegebenenfalls noch ein oder zwei weitere Heteroatome, ausgewählt aus O, N und S, enthalten kann,
R⁷,R⁸ und R⁹ unabhängig von einander für C₁₋₅-Alkyl, C₁₋₅-Alkoxy oder C₆₋₁₂-Aryl stehen
oder
zwei der Reste gemeinsam mit dem Phosphoratom, an das sie gebunden sind, einen 5-7-gliedrigen Heterocyclus bilden, der gegebenenfalls noch ein oder zwei weitere Heteroatome, ausgewählt aus O, N und S, enthalten kann.
n 2, 3 oder 4 bedeutet,
zur Herstellung von Arzneimitteln mit verbessertem Durchtritt eines pharmazeutischen Wirkstoffs durch Zell- und Organbarrieren ausgewählt aus: der Bluthirnschranke, der Placentaschranke, Muskelzellen und Leberzellen.

5. Verwendung gemäß Anspruch 4, wobei in der Formel (I)
R¹ für einen gegebenenfalls durch ein oder mehrere Fluor- oder Chloratome substituierten n-Alkylrest mit 2 bis 20 Kohlenstoffatomen, in dem eines der Kohlenstoff-Kettenglieder durch ein Sauerstoffatom ersetzt sein kann, steht.

6. Verwendung gemäß Anspruch 4 oder 5, wobei in der Formel (I)
R¹ für einen n-Alkylrest mit 5 bis 12 Kohlenstoffatomen steht.

## Claims

1. Use of compounds of the formula (I) 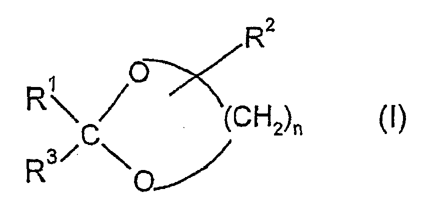 in which
R¹ represents an alkyl, alkenyl or alkynyl radical which has 2 to 30 carbon atoms and which is optionally substituted by one or more halogen atoms, where, if appropriate, one or more suitable nonadjacent carbon chain members can be replaced by oxygen atoms,
R² represents hydrogen, hydroxyl, -NH₂, -NR⁴R⁵, -N⁺(R⁴R⁵R⁶), -PR⁷R⁸, -O-P(R⁷R⁸), -P(O)R⁷R⁸, -P⁺(R⁷R⁸R⁹) or a C₁₋₅-alkyl radical which is optionally substituted by hydroxyl, C₁₋₄-alkoxy, -NH₂, mono- or di-C₁₋₄-alkylamino or a 5- to 7-membered heterocycle having up to three hetero atoms selected from among O, N and S,
R³ represents hydrogen or can have the meanings stated above for R¹,
R⁴, R⁵ and R⁶ independently of one another represent hydrogen or C₁₋₅-alkyl or two of the radicals together with the nitrogen atom to which they are bonded form a 5- to 7-membered heterocycle which can optionally additionally comprise one or two further heteroatoms selected from among 0, N and S,
R⁷, R⁸ and R⁹ independently of one another represent C₁₋₅-alkyl, C₁₋₅-alkoxy or C₆₋₁₂-aryl
or
two of the radicals together with the phosphorus atom to which they are bonded form a 5-7-membered heterocycle which can optionally additionally comprise one or two further heteroatoms selected from among O, N and S,
n denotes 2, 3 or 4,
for the preparation of pharmaceuticals with improved permeation of a pharmaceutically active substance across cell and organ barriers, the pharmaceutical being administered orally.

2. Use according to Claim 1, where, in formula (I),
R¹ represents an n-alkyl radical which has 2 to 20 carbon atoms and which is optionally substituted by one or more fluorine or chlorine atoms, where one of the carbon chain members can be replaced by an oxygen atom.

3. Use according to Claim 1 or 2, where, in formula (I),
R¹ particularly preferably represents an n-alkyl radical which has 5 to 12 carbon atoms.

4. Use of compounds of the formula (I) 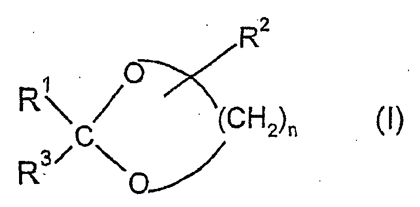 in which
R¹ represents an alkyl, alkenyl or alkynyl radical which has 2 to 30 carbon atoms and which is optionally substituted by one or more halogen atoms, where, if appropriate, one or more suitable nonadjacent carbon chain members can be replaced by oxygen atoms,
R² represents hydrogen, hydroxyl, -NH₂, -NR⁴R⁵, -N⁺(R⁴R⁵R⁶), -PR⁷R⁸, -O-P(R⁷R⁸), -P(O)R⁷R⁸, -P⁺(R⁷R⁸R⁹) or a C₁₋₅-alkyl radical which is optionally substituted by hydroxyl, C₁₋₄-alkoxy, -NH₂, mono- or di-C₁₋₄-alkylamino or a 5- to 7-membered heterocycle having up to three hetero atoms selected from among 0, N and S,
R³ represents hydrogen or can have the meanings stated above for R¹,
R⁴, R⁵ and R⁶ independently of one another represent hydrogen or C₁₋₅-alkyl or two of the radicals together with the nitrogen atom to which they are bonded form a 5- to 7-membered heterocycle which can optionally additionally comprise one or two further heteroatoms selected from among O, N and S,
R⁷, R⁸ and R⁹ independently of one another represent C₁₋₅-alkyl, C₁₋₅-alkoxy or C₆₋₁₂-aryl
or
two of the radicals together with the phosphorus atom to which they are bonded form a 5-7-membered heterocycle which can optionally additionally comprise one or two further heteroatoms selected from among O, N and S,
n denotes 2, 3 or 4,
for the preparation of pharmaceuticals with improved permeation of a pharmaceutically active substance across cell and organ barriers, selected from among: the blood-brain barrier, the placental barrier, muscle cells and liver cells.

5. Use according to Claim 4, where, in formula (I),
R¹ represents an n-alkyl radical which has 2 to 20 carbon atoms and which is optionally substituted by one or more fluorine or chlorine atoms, where one of the carbon chain members can be replaced by an oxygen atom.

6. Use according to Claim 4 or 5, where, in formula (I),
R¹ represents an n-alkyl radical which has 5 to 12 carbon atoms.

## Revendications

1. Utilisation de composés de formule (I) 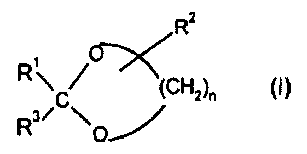 dans laquelle
R¹ représente un reste alkyle, alcényle ou alcynyle de 2 à 30 atomes de carbone, éventuellement substitué avec un ou plusieurs atomes d'halogène, un ou plusieurs membres non contigus convenables de la chaîne carbonée pouvant éventuellement être remplacés par des atomes d'oxygène,
R² représente l'hydrogène, un groupe hydroxyle, -NH₂, -NR⁴R⁵, -N⁺(R⁴R⁵R⁶), -PR⁷R⁸, -O-P(R⁷R⁸), -P(O)R⁷R⁸, -P⁺(R⁷R⁸R⁹) ou un reste alkyle en C₁ à C₅, qui est éventuellement substitué par un radical hydroxyle, alkoxy en C₁ à C₄, -NH₂, mono- ou di-(alkyle en C₁ à C₄) -amino ou par un hétérocycle pentagonal à heptagonal ayant jusqu'à trois hétéroatomes choisis parmi O, N et S,
R³ représente l'hydrogène ou peut adopter les définitions de R¹ indiquées ci-dessus,
R⁴, R⁵ et R⁶ représentent, indépendamment les uns des autres, l'hydrogène ou un reste alkyle en C₁ à C₅, ou bien deux des restes forment, conjointement avec l'atome d'azote auquel ils sont liés, un hétérocycle pentagonal à heptagonal qui peut contenir encore, le cas échéant, un ou deux autres hétéroatomes choisis parmi O, N et S,
R⁷, R⁸ et R⁹ représentent, indépendamment les uns des autres, un reste alkyle en C₁ à C₅, alkoxy en C₁ à C₅ ou aryle en C₆ à C₁₂,
ou bien
deux des restes forment, conjointement avec l'atome de phosphore auquel ils sont liés, un hétérocycle pentagonal à heptagonal qui peut encore contenir éventuellement un ou deux autres hétéroatomes choisis parmi O, N et S,
n a la valeur 2, 3 ou 4,
pour la préparation de médicaments permettant un franchissement amélioré, par une substance active pharmaceutique, de barrières de cellules et d'organes, le médicament étant administré par voie orale.

2. Utilisation suivant la revendication 1, pour laquelle, dans la formule (I),
R¹ représente un reste n-alkyle ayant 2 à 20 atomes de carbone, éventuellement substitué par un ou plusieurs atomes de fluor ou de chlore, dans lequel l'un des membres de la chaîne carbonée peut être remplacé par un atome d'oxygène.

3. Utilisation suivant la revendication 1 ou 2, pour laquelle, dans la formule (I)
R¹ représente très avantageusement un reste n-alkyle ayant 5 à 12 atomes de carbone.

4. Utilisation de composés de formule (I) 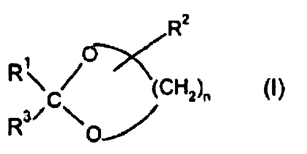 dans laquelle
R¹ représente un reste alkyle, alcényle ou alcynyle de 2 à 30 atomes de carbone, éventuellement substitué avec un ou plusieurs atomes d'halogène, un ou plusieurs membres non contigus convenables de la chaîne carbonée pouvant être remplacés par des atomes d'oxygène,
R² représente l'hydrogène, un groupe hydroxyle, -NH₂, -NR⁴R⁵, -N⁺(R⁴R⁵R⁶), -PR⁷R⁸, -O-P(R⁷R⁸), -P(O)R⁷R⁸, -P⁺(R⁷R⁸R⁹) ou un reste alkyle en C₁ à C₅, qui est éventuellement substitué par un radical hydroxyle, alkoxy en C₁ à C₄, -NH₂, mono- ou di-(alkyle en C₁ à C₄)-amino ou par un hétérocycle pentagonal à heptagonal ayant jusqu'à trois hétéroatomes choisis parmi O, N et S,
R³ représente l'hydrogène ou peut adopter les définitions de R¹ indiquées ci-dessus,
R⁴, R⁵ et R⁶ représentent, indépendamment les uns des autres, l'hydrogène ou un reste alkyle en C₁ à C₅, ou bien deux des restes forment, conjointement avec l'atome d'azote auquel ils sont liés, un hétérocycle pentagonal à heptagonal qui peut contenir encore un ou deux autres hétéroatomes choisis parmi O, N et S,
R⁷, R⁸ et R⁹ représentent, indépendamment les uns des autres, un reste alkyle en C₁ à C₅, alkoxy en C₁ à C₅ ou aryle en C₆ à C₁₂,
ou bien
deux des restes forment, conjointement avec l'atome de phosphore auquel ils sont liés, un hétérocycle pentagonal à heptagonal qui peut encore contenir éventuellement un ou deux autres hétéroatomes choisis parmi O, N et S,
n a la valeur 2, 3 ou 4,
pour la préparation de médicaments permettant un franchissement amélioré, par une substance active pharmaceutique, de barrières choisies entre la barrière hémato-encéphalique, la barrière placentaire, des cellules musculaires et des cellules hépatiques.

5. Utilisation suivant la revendication 4, pour laquelle, dans la formule (I),
R¹ représente un reste n-alkyle ayant 2 à 20 atomes de carbone, éventuellement substitué par un ou plusieurs atomes de fluor ou de chlore, dans lequel l'un des membres de la chaîne carbonée peut être remplacé par un atome d'oxygène.

6. Utilisation suivant la revendication 4 ou 5, pour laquelle, dans la formule (I),
R¹ représente un reste n-alkyle ayant 5 à 12 atomes de carbone.
